(19) Europäisches Patentamt
European Patent Office
Office européen des brevets

(11) EP 4 435 750 A1

(12) EUROPÄISCHE PATENTANMELDUNG

(43) Veröffentlichungstag:
**25.09.2024 Patentblatt 2024/39**

(21) Anmeldenummer: **24164728.8**

(22) Anmeldetag: **20.03.2024**

(51) Internationale Patentklassifikation (IPC):
***G08B 21/04*** *(2006.01)*

(52) Gemeinsame Patentklassifikation (CPC):
**G08B 21/043; G08B 21/0446**

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**
Benannte Erstreckungsstaaten:
**BA**
Benannte Validierungsstaaten:
**GE KH MA MD TN**

(30) Priorität: **20.03.2023 CH 3012023**

(71) Anmelder: **Nestor International Corporation AG**
**9050 Appenzell (CH)**

(72) Erfinder:
- **Rosenberg, Martin**
**6340 Baar (CH)**
- **Loonen, Jacobus Cornelis**
**9427 Wolfhalden (CH)**

(74) Vertreter: **Latscha Schöllhorn Partner AG**
**Grellingerstrasse 60**
**4052 Basel (CH)**

(54) **SYSTEM UND VERFAHREN ZUM ERKENNEN VON STÜRZEN**

(57) Die Erfindung ist gerichtet auf ein System (1) zur Erkennung von Stürzen von insbesondere älteren Personen, aufweisend ein tragbares Gerät (2) mit zumindest einer Sensoreinrichtung (2.2) zur Messung eines Wertes ($W_1$) für eine Sturzintensität sowie zur Messung eines Wertes ($W_2$) für eine Aktivität nach dem Sturz; eine Bedienoberfläche (3) auf welcher mittels eines ersten Reglers ($R_1$) ein erster Grenzwert ($R_{1G}$) für die Sturzintensität einstellbar ist und auf welcher mittels eines zweiten Reglers ($R_2$) ein zweiter Grenzwert ($R_{2G}$) für die Aktivität nach einem Sturz einstellbar ist, wobei das System konfiguriert ist den ersten Grenzwert ($R_{1G}$) und den zweiten Grenzwert ($R_{2G}$) an das tragbare Gerät (2) zu übermitteln. Die Erfindung ist weiterhin gerichtet auf ein entsprechendes Verfahren.

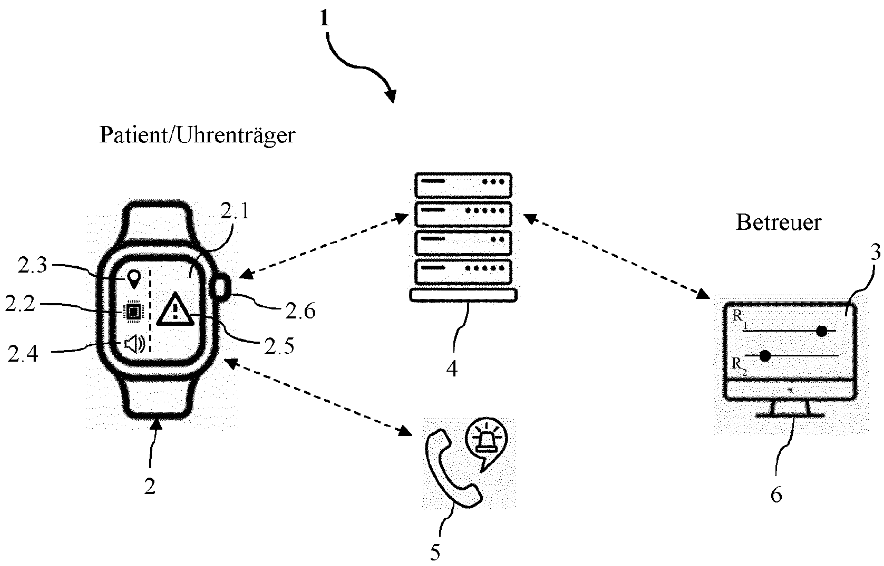


Fig. 1

EP 4 435 750 A1

## Beschreibung

### Technisches Gebiet

Die Erfindung betrifft ein System sowie ein Verfahren zur Erkennung von Stürzen von insbesondere älteren Personen. Die Erfindung findet aber beispielsweise auch Anwendung im Bereich der Arbeitssicherheit (etwa bei Alleinarbeitern) sowie im Bereich des Freizeitsports.

Derartige Systeme und Verfahren werden verwendet, um Stürze von Personen möglichst frühzeitig zu registrieren, um im Falle eines Sturzes rechtzeitig geeignete Maßnahmen treffen zu können. Solche geeigneten Maßnahmen können etwa im Aussenden eines Notrufs bestehen, wodurch für die gestürzte Person eine möglichst zeitnahe ärztliche Versorgung gewährleistet werden kann. Sturzerkennungs- bzw. Überwachungssysteme finden insbesondere bei älteren Menschen Anwendung, da diese sich nach einem Sturz häufig nicht aus eigener Kraft aufrichten bzw. einen Arzt rufen können.

Zur Sturzerkennung werden generell verschiedene Systeme und Verfahren vorgeschlagen. Hierzu zählt die Verwendung einer räumlichen Anordnung von mehreren Sensoren zur Raumüberwachung. Aktive Sensoren senden hierbei Signale aus, die von Reflektoren am Körper einer Person reflektiert werden. Auch das Auswerten von bildgebenden Verfahren zur Sturzbestimmung wurde vorgesehen. Mit diesen Verfahren ist allerdings eine aufwendige Installation der benötigten Detektoreinrichtung verbunden. Zudem bietet dieses System keine Sicherheit, sobald sich die Person im Freien bewegt, wo keine Detektoren eingerichtet sind.

Bei anderweitigen sensorbasierten Lösungen werten entsprechende Programme, etwa mit Hilfe von x, y, z Beschleunigungssensoren, Beschleunigungen und Bewegungen von tragbaren Geräten aus und analysieren diese Daten im Hinblick auf ein Sturzrisiko.

### Stand der Technik

In der US 2021/0275023 A1 werden beispielsweise ein System zur Gesundheitsfernüberwachung und ein Rahmenwerk zum Ableiten von Verhaltensdaten von tragbaren Sensoren zum Screening und Verfolgen von Krankheiten beschrieben. Das mobile/tragbare Computergerät erfasst eine Vielzahl von Metriken, die sich auf die Gesundheit, das Verhalten und das Wohlbefinden einer Person beziehen. Daten von diesen Sensoren werden aggregiert, analysiert und über ein Pflegepersonal-Dashboard, das als Smartphone-, Web- oder Smartwatch-Anwendung verfügbar ist, an eine oder mehrere Pflegekräfte gemeldet. Diese Daten bestehen aus zeitkritischen Benachrichtigungen wie Stürzen, Wandern und Extremwetterwarnungen sowie aus einem ganzheitlichen Profil des individuellen Wohlbefindens, einschließlich Stress, Schlafqualität, Fitness und Änderungen des Lebensstils. Da das mobile System im Feld verwendet wird, wird aus der Benutzerbasis eine umfangreiche Datenbank mit Verhaltensdaten generiert, die eine flexible Plattform für die Längsverfolgung und das Screening einer Vielzahl von mentalen, neurologischen und physiologischen Störungen bietet. Im Hinblick auf Stürze werden dabei Beschleunigungsmesserdaten periodisch von der Bewegungserfassungshardware abgetastet und durch den Bewegungsdetektorblock auf ähnliche Weise wie die Bewegungsdetektoreinheit ausgewertet. Einige Ausführungsformen können die Beschleunigungsmesserdaten im Speicher puffern, bevor sie von dem Bewegungsdetektorblock verarbeitet werden. Wenn die Ausführungsform einen Beschleunigungsmesser enthält, könnte die Intensität der Ablesung des dreidimensionalen Beschleunigungsmessers (x, y, z) durch die Quadratwurzel der Summe der Quadrate berechnet werden, die mit einem Schwellenwert verglichen würden, um zu bestimmen, ob die Bewegung signifikant genug ist, um einen Sturz anzuzeigen.

Dieses bekannte System bzw. Verfahren ist in seiner Ausgestaltung allerdings noch relativ statisch und führt somit zu keiner auseichend benutzerindividuellen Einstellung der für die Auslösung eines Notrufs massgeblichen Parameter.

Der vorliegenden Erfindung liegt daher die Aufgabe zu Grunde, eine Lösung zur Sturzerkennung bereitzustellen, mittels welcher möglichst rasch eine auf den jeweiligen Benutzer zugeschnittene Einstellung für eine zuverlässige Sturzerkennung gefunden wird.

### Darstellung der Erfindung

Die Aufgabe wird erfindungsgemäss durch ein System zur Erkennung von Stürzen von insbesondere älteren Personen gelöst, wie es im unabhängigen Anspruch 1 definiert ist sowie durch ein entsprechendes Verfahren, wie es im unabhängigen Anspruch 9 definiert ist. Vorteilhafte Ausführungsvarianten der Erfindung ergeben sich jeweils aus den abhängigen Ansprüchen.

Das Wesen der Erfindung besteht im Folgenden: Ein System zur Erkennung von Stürzen von insbesondere älteren Personen. Das System weist ein tragbares Gerät mit zumindest einer Sensoreinrichtung zur Messung eines Wertes für eine Sturzintensität sowie zur Messung eines Wertes für eine Aktivität nach dem Sturz auf. Weiterhin umfasst das System eine Bedienoberfläche, auf welcher mittels eines ersten Reglers ein erster Grenzwert für die Sturzintensität einstellbar ist und auf welcher mittels eines zweiten Reglers ein zweiter Grenzwert für die Aktivität nach einem Sturz einstellbar ist. Das System ist dabei so konfiguriert, den ersten Grenzwert und den zweiten Grenzwert an das tragbare Gerät zu übermitteln.

Der Begriff "tragbares Gerät" umfasst vorliegend etwa eine Uhr bzw. eine Sturzuhr, einen Anhänger oder ein Armband (oder eine ähnliche geeignete Hardware), welches die betreffende Person am Körper trägt. Mittels des tragbaren Geräts werden sowohl die Sturzintensität als auch die Aktivität nach dem Sturz gemessen.

Die "Sturzintensität" ist ein Mass für die Schwere eines potenziellen Sturzes; sie wird vielfach aber auch über unterschiedliche Aktivitäten simuliert wie beispielsweise bei der Verwendung von Rollatoren auf Kopfsteinpflaster, beim Teppichklopfen, bei Handwerksarbeiten oder etwa beim Anstossen eines Armes an Ecken oder Kanten im Haushalt. Deshalb ist es entscheidend, dass das System nicht bei all diesen Tätigkeiten einen Notruf auslöst.

Zu diesem Zweck wird eine unmittelbare nachgelagerte Messung zur Feststellung der "Aktivität nach dem Sturz" durchgeführt. Bewegt sich etwa der Arm der betreffenden Person weiterhin, wird der Alarmprozess abgebrochen. Man geht davon aus, dass wenn sich die betreffende Person nach einem Sturz noch bewegen kann, sie auch die Möglichkeit hätte, den Notruf über einen Knopfdruck selbst auszulösen.

Der Begriff "Bedienoberfläche" umfasst vorliegend beispielsweise den Bildschirm eines Desktop Computers, eines Laptop Computers, eines Tablet Computers oder eines Smartphones oder dergleichen.

Vorzugsweise ist die Bedienoberfläche eine webbasierte Bedienoberfläche, auf welcher der erste Grenzwert stufenlos mittels des ersten Reglers einstellbar ist und der zweite Grenzwert stufenlos mittels des zweiten Reglers einstellbar ist. Der Begriff "webbasiert" ist vorliegend so zu verstehen, dass das Programm (bzw. die Software) zur Steuerung und Überwachung Systems (bzw. des Verfahrens) nicht auf einem Rechner installiert werden muss, sondern lediglich eine Internetverbindung oder aber eine Mobilfunkverbindung nötig ist, um das Programm, welches etwa auf einem Server liegt, zu nutzen. Über einen Browser kann das (webbasierte) Programm online aufgerufen werden. Durch die stufenlose Einstellbarkeit der beiden Regler kann eine besonders dynamische und personenindividuelle Einstellung vorgenommen werden. Anstelle einer manuellen Einstellung im Benutzerkonto kann das System so konfiguriert werden, dass aufgrund von Bewegungsmustern mittels von Künstlicher Intelligenz (KI) das System die optimale Einstellung selber vornimmt.

Vorzugsweise umfasst das System weiterhin einen Server, welcher die auf der Bedienoberfläche eingestellten Grenzwerte an das tragbare Gerät (per Datenübertragung) übermittelt, wobei vorzugsweise auf dem Server ein Programm zur Regelung und Steuerung des Systems liegt. Auf diese Weise kann eine besonders gute Zugänglichkeit und Sicherheit des Systems erreicht werden.

Vorzugsweise umfasst die Sensoreinrichtung einen Beschleunigungsmesser. Diese Ausgestaltung hat sich als besonders effektiv und robust in der Anwendung herausgestellt. Allerdings können im Rahmen der vorliegenden Erfindung zahlreiche weitere Sensoreinrichtungen (jeweils allein oder in Kombination) Anwendung finden, von denen einige nachfolgend beispielhaft aufgeführt und erläutert sind.

Beschleunigungssensor(en): Ein Beschleunigungssensor misst die Beschleunigung und kann verwendet werden, um Bewegungen des Geräts zu erfassen und Stürze zu erkennen.

Gyroskop(e): Ein Gyroskop misst die Rotationsgeschwindigkeit und -richtung und kann ebenfalls zur Erkennung von Bewegungen und Stürzen verwendet werden.

Barometer: Ein Barometer misst den Luftdruck und kann zur Erkennung von Änderungen in der Höhe oder des Aufpralls verwendet werden.

GPS-Sensor(en): Ein GPS-Sensor kann zur Bestimmung des Standorts und der Geschwindigkeit des Benutzers verwendet werden und somit indirekt auch zur Sturzerkennung beitragen.

PPG-Sensor(en): Ein PPG-Sensor (Photoplethysmography) kann zur Messung der Herzfrequenz verwendet werden, aber auch zur Messung der Bewegungen des Körpers, die bei einem Sturz auftreten können.

Infrarot-Sensor(en): Ein Infrarot-Sensor kann verwendet werden, um die Umgebung des Benutzers zu erkennen und Stürze durch Hindernisse oder andere Objekte zu erkennen.

Ultraschallsensor(en): Ein Ultraschallsensor kann verwendet werden, um die Entfernung zu Hindernissen und anderen Objekten zu messen und so Stürze durch Kollisionen mit Hindernissen zu erkennen.

Magnetometer: Ein Magnetometer misst das Magnetfeld und kann zur Erkennung von Bewegungen und Richtungsänderungen des Geräts verwendet werden.

Temperatursensor(en): Ein Temperatursensor kann verwendet werden, um den Körper und die Umgebungstemperatur zu messen und so auf Veränderungen und Stürze zu schließen.

Mikrofon(e): Ein Mikrofon kann verwendet werden, um Geräusche und Vibrationen zu erfassen, die bei einem Sturz auftreten können.

Näherungssensor(en): Ein Näherungssensor kann zur Erkennung von Hindernissen und anderen Objekten in der Nähe des Benutzers verwendet werden, um Stürze durch Kollisionen mit Hindernissen zu erkennen.

Vorzugsweise ist der Beschleunigungsmesser ein dreidimensionaler Beschleunigungsmesser, welcher insbesondere einen Wert für die Intensität eines Sturzes sowie einen Wert für die Aktivität nach einem Sturz anhand der Formel

$$W_{1,2} = \sqrt[2]{x^2 + y^2 + z^2}$$

bestimmt.

Je höher diese Werte sind, umso stärker ist entsprechend die Beschleunigung. Das System liest hierzu vom Beschleunigungsmesser drei Werte x, y und z ab (d.h. dreidimensional über drei Achsen), die in Metern pro Sekunde dargestellt sind. Je höher der errechnete Wert ist, umso stärker ist die Beschleunigung welche durch den in (z.B. der Sturzuhr eingebauten) Beschleunigungsmesser gemessen wurde. Die Werte können grundsätzlich aber auch über mehr oder weniger Achsen errechnet werden. Die dreidimensionale Messung bietet jedoch eine höhere Sicherheit in Bezug auf Beschleunigungsmessungen in alle Richtungen (d.h. die vertikale, horizontale und diagonale Sturzrichtung).

Vorzugsweise umfasst das System weiterhin eine Notrufzentrale bzw. Notrufnummern, welche von dem tragbaren Gerät kontaktierbar ist/sind. Im Falle eines Sturzereignisses, nach welchem sich die betreffende Person nicht mehr bzw. nicht mehr ausreichend bewegt (d.h. der entsprechend ausgelöste Alarm wurde von der betreffenden Person nicht storniert), kontaktiert die Sturzuhr automatisch eskalierend jede einzelne im System hinterlegte Notrufnummer und meldet unmittelbar an diese via SMS die Koordinaten zur Ortung der Sturzuhr. Gleichzeitig wird eine Sprachverbindung mit der betreffenden Person hergestellt.

Ein weiterer Aspekt der Erfindung umfasst: Ein Verfahren zur Erkennung von Stürzen von insbesondere älteren Personen. Das Verfahren umfasst die folgenden Schritte: Einstellen eines Grenzwertes für eine Sturzintensität sowie eines Grenzwertes für eine Aktivität nach dem Sturz auf einer Bedienoberfläche; Messen eines Wertes für die Sturzintensität mit einem tragbaren Gerät; Vergleichen des Messwerts für die Sturzintensität mit dem eingestellten Grenzwert für die Sturzintensität; und bei Überschreitung des Grenzwertes weiterhin: Messen eines Wertes für die Aktivität nach dem Sturz nach vorbestimmter Zeitdauer mit dem tragbaren Gerät; Vergleichen des Messwertes für die Aktivität nach dem Sturz mit dem eingestellten Grenzwert für die Aktivität nach dem Sturz; und bei Unterschreitung des Grenzwertes weiterhin: Auslösen eines Alarms; Abwarten, ob der ausgelöste Alarm innerhalb einer vorbestimmten Zeitdauer storniert wird; und wenn der Alarm nicht storniert wird weiterhin: Auslösen eines Notrufes durch das tragbare Gerät (bzw. Kontaktieren der Notrufzentrale).

Vorzugsweise wird bei einer Überschreitung des Grenzwertes für die Sturzintensität der gemessene Wert für die Sturzintensität gespeichert, d.h. etwa in einem Zwischenspeicher (Buffer) des Beschleunigungsmessers bzw. des tragbaren Geräts und/oder auf dem Server. Die Speicherung dieser Werte ist für eine etwaige spätere Selbstkorrektur des Systems mittels eines iterativen Prozesses zur Ermittlung der jeweils optimalen Grenzwerte für die betreffende Person erforderlich.

Vorzugsweise beträgt die vorbestimmte Zeitdauer, nach welcher der Wert für die Aktivität nach dem Sturz gemessen wird, zwischen etwa 1 Sekunde und etwa 5 Sekunden, vorzugsweise zwischen etwa 2 Sekunden und etwa 4 Sekunden und weiter vorzugsweise etwa 3 Sekunden. Dies hat sich in der Praxis als besonders effizient erwiesen. Diese Intervallmessung kann über das Benutzerkonto eingestellt werden, um auch im Falle von Alleinarbeitern eine Sicherheit zu bieten, welche den Modus des "Totmanns" benötigen. In diesen Fällen kann dies, je nach Branche, bis auf mehrere Minuten eingestellt werden. Totmannschaltungen dienen der Arbeitssicherheit an Einzelarbeitsplätzen oder an gefährlichen Maschinen und sind häufig gesetzlich, zumindest aber versicherungsrechtlich vorgeschrieben. Sie reagieren etwa auf Bewegungslosigkeit, waagerechte Körperlage oder Schlaf.

Vorzugsweise beträgt des Weiteren die vorbestimmte Zeitdauer, innerhalb welcher die betreffende Person den Alarm selbst stornieren kann, zwischen etwa 10 Sekunden und etwa 30 Sekunden, vorzugsweise zwischen etwa 12 Sekunden und etwa 25 Sekunden und weiter vorzugsweise etwa 15 Sekunden beträgt.

Vorzugsweise endet der Prozess, wenn der Grenzwert für die Sturzintensität nicht überschritten wurde. In diesem Fall liegt für die betreffende Person kein relevantes Sturzereignis vor. Die Einstellung richtet sich dabei nach dem Gesundheitszustand bzw. dem Fitness-Level der jeweiligen Person. Für Personen, welche ggf. noch Sport betreiben (z.B. Wandern oder Nordic Walking), wird man den Grenzwert für die Stossintensität höher wählen als für Personen die sich vorwiegend nur noch im Haus aufhalten.

Vorzugsweise wird kein Alarm ausgelöst, wenn der Grenzwert für die Aktivität nach dem Sturz nicht unterschritten wird. Der Hintergedanke hierbei ist wiederum, dass wenn sich die betreffende Person nach einem Sturz noch bewegen kann, sie auch die Möglichkeit hätte, den Notruf über beispielsweise einen Knopfdruck (an der Uhr oder auf dem Display) selbst auszulösen.

Vorzugsweise wird nach einem Stornieren des Alarms der Prozess beendet und die Prozessdaten werden gesammelt. Auf diese Weise soll entsprechend das unnötige Auslösen eines Notrufs verhindert werden und zudem sollen sämtliche Daten für eine spätere Selbstkorrektur des Systems mittels des vorerwähnten iterativen Prozesses zur Ermittlung der jeweils optimalen Grenzwerte für die betreffende Person bereitgestellt werden.

Ein weiterer Aspekt der vorliegenden Erfindung umfasst zudem ein Computerprogrammprodukt, welches Befehle umfasst, die bei der Ausführung des Programms durch einen Computer diesen veranlassen, das vorbeschriebene Verfahren auszuführen. Dieses Computerprogrammprodukt kann insbesondere auf dem Server liegen.

Mit Blick auf die vorbeschriebene Erfindung soll an dieser Stelle noch hervorgehoben werden, dass nur wenn ein Sturzereignis zuverlässig als solches erkannt werden kann, die betreffende Person motiviert sein wird, ihre Sturzuhr (oder ein anderweitiges Gerät) auch immer zu tragen. Ist dies nicht der Fall, schaltet der Uhrenträger respektive der Betreuer den automatischen Sturzalarm aus. Damit erhöht sich das Risiko von schweren Folgen nach einem Sturz, wenn der Gestürzte nicht unmittelbare Hilfe bekommt. Denkt man hier etwa an Schlaganfälle, Herzschwächen, Thrombosen, Schwindelanfälle, Stolperstürze, etc., so können die Folgen bei nicht rechtzeitiger ärztlicher Behandlung hohe Rehabilitationskosten verursachen, oder bei nicht rechtzeitigem Auffinden sogar den Tod der betreffenden Person bedeuten.

Kurze Beschreibung der Zeichnungen

Weitere vorteilhafte Ausgestaltungen der Erfindung ergeben sich aus der nachfolgenden Beschreibung von Ausführungsbeispielen der Erfindung mit Hilfe der schematischen Zeichnung. Insbesondere wird im Folgenden das erfindungsgemässe System/Verfahren unter Bezugnahme auf die beigefügten Zeichnungen anhand von Ausführungsbeispielen detaillierter beschrieben. Es zeigen:

Fig. 1: eine schematische Darstellung eines erfindungsgemässen Sturzerkennungssystems;

Fig. 2: eine schematische Darstellung des ersten Reglers und des zweiten Reglers auf einer Bedienoberfläche eines erfindungsgemässen Sturzerkennungssystems; und

Fig. 3 ein Flussdiagramm, in welchem der Ablauf eines erfindungsgemässen Sturzerkennungsverfahrens schematisch dargestellt ist.

Weg(e) zur Ausführung der Erfindung

Bestimmte Ausdrücke werden in der folgenden Beschreibung ggf. aus praktischen Gründen verwendet und sind nicht einschränkend zu verstehen. Die Wörter "rechts", "links", "unten" und "oben" bezeichnen Richtungen in der Zeichnung, auf die Bezug genommen wird. Die Ausdrücke "nach innen", "nach aussen" "unterhalb", "oberhalb", "links", "rechts" oder ähnliche werden zur Beschreibung der Anordnung bezeichneter Teile zueinander, der Bewegung bezeichneter Teile zueinander und der Richtungen hin zum oder weg vom geometrischen Mittelpunkt der Erfindung sowie benannter Teile derselben wie in den Figuren dargestellt verwendet. Diese räumlichen Relativangaben umfassen auch andere Positionen und Ausrichtungen als die in den Figuren dargestellten. Zum Beispiel wenn ein in den Figuren dargestelltes Teil umgedreht wird, sind Elemente oder Merkmale, die als "unterhalb" beschrieben sind, dann "oberhalb". Die Terminologie umfasst die oben ausdrücklich erwähnten Wörter, Ableitungen von denselben und Wörter ähnlicher Bedeutung.

Um Wiederholungen in den Figuren und der zugehörigen Beschreibung der verschiedenen Aspekte und Ausführungsbeispiele zu vermeiden, sollen bestimmte Merkmale als gemeinsam für verschieden Aspekte und Ausführungsbeispiele verstanden werden. Das Weglassen eines Aspekts in der Beschreibung oder einer Figur lässt nicht darauf schliessen, dass dieser Aspekt in dem zugehörigen Ausführungsbeispiel fehlt. Vielmehr kann ein solches Weglassen der Klarheit und dem Verhindern von Wiederholungen dienen. In diesem Zusammenhang gilt für die gesamte weitere Beschreibung folgende Festlegung: Sind in einer Figur zum Zweck zeichnerischer Eindeutigkeit Bezugszeichen enthalten, aber im unmittelbar zugehörigen Beschreibungstext nicht erwähnt, so wird auf deren Erläuterung in vorangehenden Figurenbeschreibungen Bezug genommen. Sind ausserdem im unmittelbar zu einer Figur gehörigen Beschreibungstext Bezugszeichen erwähnt, die in der zugehörigen Figur nicht enthalten sind, so wird auf die vorangehenden und nachstehenden Figuren verwiesen. Ähnliche Bezugszeichen in zwei oder mehreren Figuren stehen für ähnliche oder gleiche Elemente.

In **Fig. 1** wird ein erfindungsgemässes System 1 zur Erkennung von Stürzen gezeigt. Auf der Betreuerseite befindet sich die webbasierte Bedienoberfläche 3, über welche der oder die Betreuer mittels des ersten Reglers $R_1$ und des zweiten Reglers $R_2$ die entsprechenden Grenzwerte für die Sturzintensität und für die Aktivität nach dem Sturz einstellen können. Die webbasierte Oberfläche 3 bzw. das entsprechende End- bzw. Betreuergerät (z.B. Computer, Laptop, Smartphone oder Tablet) ist über das Internet oder in sonstiger geeigneter Art und Weise bidirektional mit dem Server 4 verbunden, auf welchem vorzugsweise das Programm bzw. die Software zur Steuerung und Überwachung des Systems 1 bzw. des Verfahrens liegt. Der Server 4 seinerseits ist bidirektional mit dem tragbaren Gerät, hier in From einer Sturzuhr 2, auf der Patienten- bzw. Uhrenträgerseite verbunden. Die Verbindung zwischen dem Server und dem tragbaren Gerät 2 erfolgt ebenfalls über das Internet (oder in sonstiger geeigneter Art und Weise).

Das tragbare Gerät 2 umfasst generell ein Display 2.1 auf welchem vorliegend ein Alarm-Icon 2.5 angezeigt wird, was üblicherweise von einem entsprechenden akustischen Signal aus der Mikrophon/Lautsprecher-Einheit 2.4 begleitet wird. Dieser Alarm kann vom Patienten durch Antippen des Alarm-Icons 2.5 auf dem Display 2.1 storniert werden. Alternativ könnte der Alarm auch durch Betätigen des Druckknopfs 2.6 am Gehäuserand storniert werden; dieser kann aber auch als zum Auslösen eines Notrufs durch den Patienten vorgesehen sein, d.h. für den Fall das der Patient noch selber in der Lage ist, einen solchen nach einem Sturz (oder in einer anderweitigen Notsituation) auszulösen.

Das tragbare Gerät 2 ist generell bidirektional mit einer Notrufzentrale 5 und/oder der Rufnummer von Angehörigen verbunden, an welche das tragbare Gerät 2 im Falle eines Notrufs eine Benachrichtigung per SMS schickt zusammen mit beispielsweise den GPS-Koordinaten des Patienten. Gleichzeitig wird ein Telefonanruf an die im System gespeicherten (Not-)Rufnummern gestartet. Das System erlaubt die Speicherung von unzähligen Not-(Rufnummern). Diese werden bei einem Notfall eskalierend angerufen, sofern die angerufene Notfall-Telefonnnummer den Anruf nicht innerhalb einer voreingestellten Zeitdauer (z.B. 15 Sekunden) entgegennimmt. Das tragbare Gerät 2 umfasst entsprechend eine GPS-Einheit 2.3, einen Beschleunigungsmesser 2.2 sowie eine Mikrophon/Lautsprecher-Einheit 2.4 (diese Komponenten sind in der Fig. 1 lediglich schematisch links neben der gestrichelten Linie dargestellt und befinden sich im Inneren des Geräts). Grundsätzlich kann das tragbare Gerät 2 etwa auch über eine Bluetooth-Einheit verfügen, über welche etwa die Grenzwerteinstellungen übermittelt werden. Die Daten vom Server 4 können auch über SMS an das tragbare Gerät 2 übertragen werden. Über die Mikrophon/Lautsprecher-Einheit 2.4 kann, neben der Aussendung eines akustischen Signals, ggf. auch eine telefonische Kontaktaufnahme mit dem Patienten erfolgen.

In **Fig. 2** wird eine beispielhafte Bedienoberfläche 3 für ein erfindungsgemässes System 1 zur Erkennung von Stürzen illustriert. Der erste Regler $R_1$ ist dabei auf einen Grenzwert $R_{1G}$ von 4599 auf einer Skala von 1500 bis 5000 eingestellt, d.h. dass die Auslösung eines Alarms erst bei einer relativ hohen Beschleunigung bzw. einer hohen (Sturz-) Intensität erfolgt, so dass das System insoweit relativ unempfindlich ist. Der zweite Regler $R_2$ ist hier auf einen Grenzwert $R_{2G}$ von 1300 auf einer Skala von 1000 bis 2000 eingestellt, d.h. dass eine Stornierung eines Alarms (welcher nach einer Überschreitung des Grenzwertes $R_{1G}$ ausgelöst wurde) bereits bei einer relativ kleinen Beschleunigung bzw. einer geringfügigen Aktivität nach dem Sturz erfolgt. Der Minimalwert liegt bei knapp 1000, was die kleinsten Bewegungen bedeutet. Auf diese Weise kann eine auf den jeweiligen Benutzer (bzw. dessen Lebensumstände, Gesundheitszustand und/oder Fitness-Level) zugeschnittene, dynamische Einstellung der beiden Grenzwerte $R_{1G}$ bzw. $R_{2G}$ vorgenommen werden, mit welcher eine Fehlauslösung eines Notrufs in der Praxis nahezu eliminiert wird.

Anhand von **Fig. 3** wird der Ablauf eines erfindungsgemässen Sturzerkennungsverfahrens mittels eines Flussdiagramms veranschaulicht. Zunächst wird mittels des ersten Reglers $R_1$ ein Grenzwert $R_{1G}$ für die Sturzintensität eingestellt (z.B. wie in Fig. 2 ein Wert von 4599) sowie mittels des zweiten Reglers $R_2$ ein zweiter Grenzwert $R_{2G}$ für die Aktivität nach dem Sturz (z.B. wie in Fig. 2 ein Wert von 1300). Überschreitet nun der mit dem Sensor bzw. dem Beschleunigungsmesser 2.2 gemessene Wert $W_1$ für die Sturzintensität den eingestellten Grenzwert $R_{1G}$ so wird dieser Wert $W_1$ in einem Zwischenspeicher (Buffer) gespeichert, bis die Messungen/Berechnungen bezüglich der Aktivität nach dem Sturz durchgeführt sind. Wird der Grenzwert $R_{1G}$ nicht überschritten, endet der Prozess.

Die entsprechende (zweite) Beschleunigungsmessung bezüglich der Aktivität nach dem Sturz erfolgt vorzugsweise nach einer Zeit $t_1$ von etwa 3 Sekunden nach dem Sturz und liefert einen Wert $W_2$. Sollte der Wert $W_2$ für die Aktivität nach dem Sturz unterhalb des eingestellten Grenzwertes $R_{2G}$ sein, wird ein Sturzalarm ausgelöst. Nach der Auslösung des Sturzalarms erfolgen auf der Uhr etwa ein akustisches Signal sowie eine Bildschirmdarstellung, dass ein Alarm ausgelöst wurde (vgl. Fig. 1). Der Uhrenträger kann nun innerhalb eines Zeitraums $t_2$ von etwa 15 Sekunden den Alarm stornieren oder ihn laufen zu lassen. Wird der Sturzalarm nicht storniert, telefoniert die Uhr automatisch eskalierend an jede einzelne im System hinterlegte Notruftelefonnummer und meldet unmittelbar an sie via SMS die GPS-Daten zur Ortung der Uhr bzw. des Patienten.

Wird anderseits der Sturzalarm storniert, endet der Prozess und die entsprechenden Daten werden gesammelt und einem iterativen Prozess zugeführt, welcher bei mehrmaligen "Fehlalarmen" mittels künstlicher Intelligenz (KI) eine Selbstkorrektur des Systems durchführt, bis die für den einzelnen Benutzer optimalen Grenzwerte erreicht sind. Hiermit wird eine fast punktgenaue Grenzwerteinstellung möglich. Das System lernt somit die Verhaltensmuster des jeweiligen Uhrenträgers, wodurch Fehlauslösungen von Notrufen nahezu vollständig ausgeschlossen werden können.

Es sei an dieser Stelle nochmals darauf hingewiesen, dass eine Intensität eines Sturzes vielfach über unterschiedliche Aktivitäten simuliert wird wie z.B. die Verwendung von Rollatoren über Pflastersteine, beim Teppichklopfen, bei Handwerksarbeiten oder beim Anstossen des Armes an Ecken und Kanten im Haushalt etc. Deshalb ist es entscheidend, dass das System nicht bei all diesen Tätigkeiten Sturzalarme auslöst. Zu diesem Zweck wird die unmittelbare nachgelagerte Messung der Aktivität nach dem Sturz durchgeführt. Bewegt sich der Arm weiterhin, wird der Alarm abgebrochen. Man geht davon aus, wenn sich nach einem Sturz die Person noch bewegen kann, sie die Möglichkeit hätte den Notruf über einen Knopfdruck selbst auszulösen.

Die vorliegende Offenbarung umfasst auch Ausführungsformen mit jeglicher Kombination von Merkmalen, die vorstehend oder nachfolgend zu verschiedenen Ausführungsformen genannt oder gezeigt sind. Sie umfasst ebenfalls einzelne Merkmale in den Figuren, auch wenn sie dort im Zusammenhang mit anderen Merkmalen gezeigt sind und/oder vorstehend oder nachfolgend nicht genannt sind. Auch können die in den Figuren und der Beschreibung beschriebenen Alternativen von Ausführungsformen und einzelne Alternativen deren Merkmale vom Erfindungsgegenstand beziehungsweise von den offenbarten Gegenständen ausgeschlossen sein. Die Offenbarung umfasst Ausführungsformen, die ausschliesslich die in den Ansprüchen beziehungsweise in den Ausführungsbeispielen beschriebenen Merkmale umfasst sowie auch solche, die zusätzliche andere Merkmale umfassen.

Im Weiteren schliesst der Ausdruck "umfassen" und Ableitungen davon andere Elemente oder Schritte nicht aus. Ebenfalls schliesst der unbestimmte Artikel "ein" bzw. "eine" und Ableitungen davon eine Vielzahl nicht aus. Die Funktionen mehrerer in den Ansprüchen aufgeführter Merkmale können durch eine Einheit beziehungsweise einen Schritt erfüllt sein. Die Begriffe "im Wesentlichen", "etwa", "ungefähr" und dergleichen in Verbindung mit einer Eigenschaft beziehungsweise einem Wert definieren insbesondere auch genau die Eigenschaft beziehungsweise genau den Wert. Die Begriffe "etwa" und "ungefähr" im Zusammenhang mit einem gegebenen Zahlenwert oder -bereich kann sich auf einen Wert beziehungsweise Bereich beziehen, der innerhalb 20%, innerhalb 10%, innerhalb 5% oder innerhalb 2% des gegebenen Werts beziehungsweise Bereichs liegt.

Liste der Bezugszeichen-.

| | |
|---|---|
| 1 | Sturzüberwachungssystem |
| 2 | tragbares Gerät (z.B. Sturzuhr) |
| 2.1 | Display |
| 2.2 | Sensoreinrichtung (Beschleunigungsmesser) |
| 2.3 | GPS-Einheit |
| 2.4 | Mikrophon/Lautsprecher-Einheit |
| 2.5 | Alarmanzeige auf Display |
| 2.6 | Druckknopf |
| 3 | Bedienoberfläche (webbasiert) |
| 4 | Server |
| 5 | Notrufzentrale/Angehörige, etc. (Notruf-Telefonnummern) |
| 6 | Betreuergerät (Computer, Laptop, Tablet, Handy, etc.) |
| $R_1$ | erster Regler (Sturzintensität) |
| $R_{1G}$ | Grenzwert für Sturzintensität |
| $R_2$ | Regler (Aktivität nach Sturz) |
| $R_{2G}$ | Grenzwert für Aktivität nach Sturz |
| $W_1$ | Messwert für Sturzintensität |
| $W_2$ | Messwert für Aktivität nach Sturz |

## Patentansprüche

1. System (1) zur Erkennung von Stürzen von insbesondere älteren Personen, aufweisend

   ein tragbares Gerät (2) mit zumindest einer Sensoreinrichtung (2.2) zur Messung eines Wertes ($W_1$) für eine Sturzintensität sowie zur Messung eines Wertes ($W_2$) für eine Aktivität nach dem Sturz;
   eine Bedienoberfläche (3) auf welcher mittels eines ersten Reglers ($R_1$) ein erster Grenzwert ($R_{1G}$) für die Sturzintensität einstellbar ist und auf welcher mittels eines zweiten Reglers ($R_2$) ein zweiter Grenzwert ($R_{2G}$) für die Aktivität nach einem Sturz einstellbar ist, wobei
   das System konfiguriert ist den ersten Grenzwert ($R_{1G}$) und den zweiten Grenzwert ($R_{2G}$) an das tragbare Gerät (2) zu übermitteln.

2. System (1) zur Erkennung von Stürzen gemäss Anspruch 1, wobei die Bedienoberfläche (3) eine webbasierte Bedienoberfläche ist, auf welcher der erste Grenzwert ($R_{1G}$) stufenlos mittels des ersten Reglers ($R_1$) einstellbar ist und der zweite Grenzwert ($R_{2G}$) stufenlos mittels des zweiten Reglers ($R_2$) einstellbar ist.

3. System (2) zur Erkennung von Stürzen gemäss Anspruch 1 oder 2, wobei das System weiterhin einen Server (3) umfasst, welcher die auf der Bedienoberfläche (3) eingestellten Grenzwerte ($R_{1G}$; $R_{2G}$) an das tragbare Gerät (2) übermittelt, wobei vorzugsweise auf dem Server (3) ein Programm zur Regelung und Steuerung des Systems liegt.

4. System (1) zur Erkennung von Stürzen gemäss einem der vorhergehenden Ansprüche, wobei das tragbare Gerät (2) eine Uhr, ein Anhänger oder ein Armband ist.

5. System (1) zur Erkennung von Stürzen gemäss einem der vorhergehenden Ansprüche, wobei die Sensoreinrichtung (2.2) einen Beschleunigungsmesser umfasst.

6. System (1) zur Erkennung von Stürzen gemäss Anspruch 5, wobei der Beschleunigungsmesser ein dreidimensionaler Beschleunigungsmesser ist.

7. System (1) zur Erkennung von Stürzen gemäss Anspruch 6, wobei der dreidimensionale Beschleunigungsmesser einen Wert ($W_1$) für die Intensität eines Sturzes sowie einen Wert ($W_2$) für die Aktivität nach einem Sturz anhand der Formel

$$W_{1,2} = \sqrt[2]{x^2 + y^2 + z^2}$$

   bestimmt.

8. System (1) zur Erkennung von Stürzen gemäss einem der vorhergehenden Ansprüche, wobei das System weiterhin eine Notrufzentrale/Notrufnummern (5) umfasst, welche von dem tragbaren Gerät kontaktierbar ist.

9. Verfahren zur Erkennung von Stürzen von insbesondere älteren Personen, welches die folgenden Schritte umfasst:

   Einstellen eines Grenzwertes ($R_{1G}$) für eine Sturzintensität sowie eines Grenzwertes ($R_{2G}$) für eine Aktivität nach dem Sturz auf einer Bedienoberfläche (3);
   Messen eines Wertes ($W_1$) für die Sturzintensität mit einem tragbaren Gerät (2);
   Vergleichen des Messwerts ($W_1$) für die Sturzintensität mit dem eingestellten Grenzwert ($R_{1G}$) für die Sturzintensität;
   bei Überschreitung des Grenzwertes ($R_{1G}$):

      Messen eines Wertes ($W_2$) für die Aktivität

nach dem Sturz nach vorbestimmter Zeitdauer ($t_1$) mit dem tragbaren Gerät (2);
Vergleichen des Messwertes ($W_2$) für die Aktivität nach dem Sturz mit dem eingestellten Grenzwert ($R_{2G}$) für die Aktivität nach dem Sturz;
bei Unterschreitung des Grenzwertes ($R_{2G}$):

Auslösen eines Alarms;
Abwarten, ob der ausgelöste Alarm innerhalb einer vorbestimmten Zeitdauer ($t_2$) storniert wird;
wenn Alarm nicht storniert wird:
Auslösen eines Notrufes durch das tragbare Gerät (2).

**10.** Verfahren zur Erkennung von Stürzen gemäss Anspruch 9, wobei bei einer Überschreitung des Grenzwertes ($R_{1G}$) der gemessene Wert ($W_1$) für die Sturzintensität gespeichert wird.

**11.** Verfahren zur Erkennung von Stürzen gemäss Anspruch 9 oder 10, wobei die vorbestimmte Zeitdauer ($t_1$) zwischen etwa 1 Sekunde und etwa 5 Sekunden, vorzugsweise zwischen etwa 2 Sekunden und etwa 4 Sekunden und weiter vorzugsweise etwa 3 Sekunden beträgt.

**12.** Verfahren zur Erkennung von Stürzen gemäss einem der Ansprüche 9 bis 11, wobei die vorbestimmte Zeitdauer ($t_2$) zwischen etwa 10 Sekunden und etwa 30 Sekunden, vorzugsweise zwischen etwa 12 Sekunden und etwa 25 Sekunden und weiter vorzugsweise etwa 15 Sekunden beträgt.

**13.** Verfahren zur Erkennung von Stürzen gemäss einem der Ansprüche 9 bis 12, wobei der Prozess endet wenn der Grenzwert ($R_{1G}$) nicht überschritten wurde.

**14.** Verfahren zur Erkennung von Stürzen nach einem der Ansprüche 9 bis 13, wobei kein Alarm ausgelöst wird, wenn der Grenzwert ($R_{2G}$) nicht unterschritten wird.

**15.** Verfahren zur Erkennung von Stürzen nach einem der Ansprüche 9 bis 14, wobei nach einem Stornieren des Alarms der Prozess beendet wird und die Prozessdaten gesammelt werden.

**16.** Verfahren zur Erkennung von Stürzen nach Anspruch 15, wobei nach zwei oder mehrmaligem Stornieren eines Alarms mittels eines iterativen Prozesses die optimalen Grenzwerte für die jeweilige Person ermittelt werden.

**17.** Computerprogrammprodukt, umfassend Befehle, die bei der Ausführung des Programms durch einen Computer diesen veranlassen, das Verfahren gemäss einem der Ansprüche 9 bis 16 auszuführen.

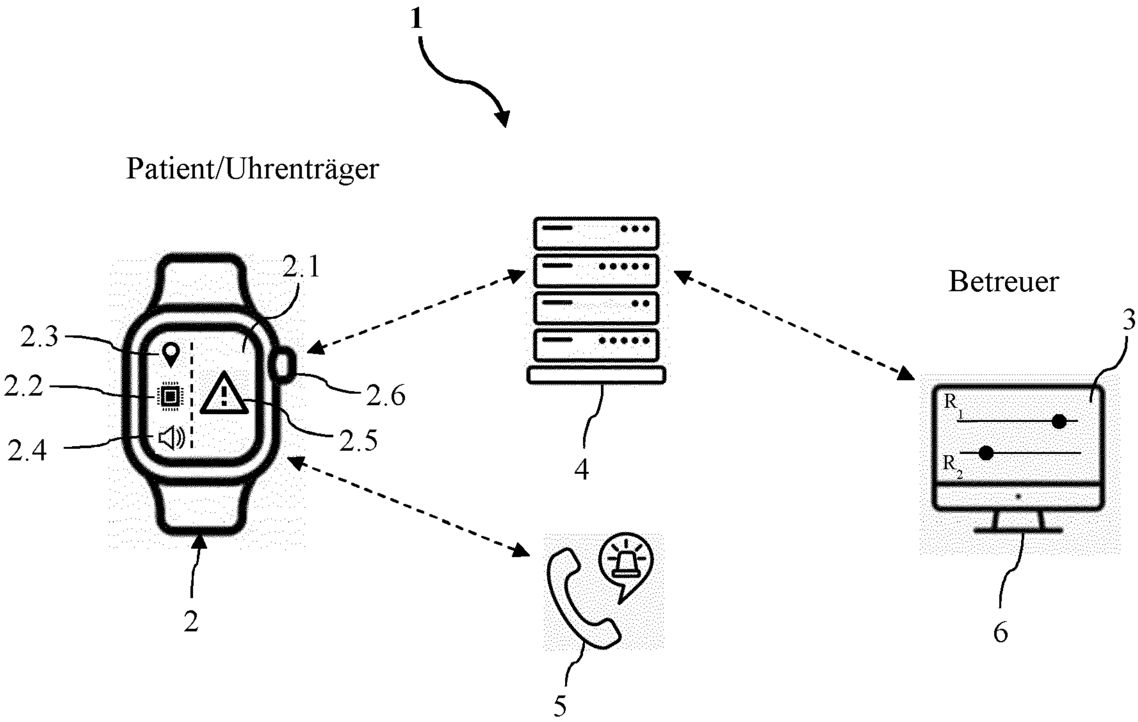
1
Patient/Uhrenträger
2.1
2.3
2.2
2.4
2.6
2.5
2
4
5
Betreuer
3
$R_1$
$R_2$
6

Fig. 1

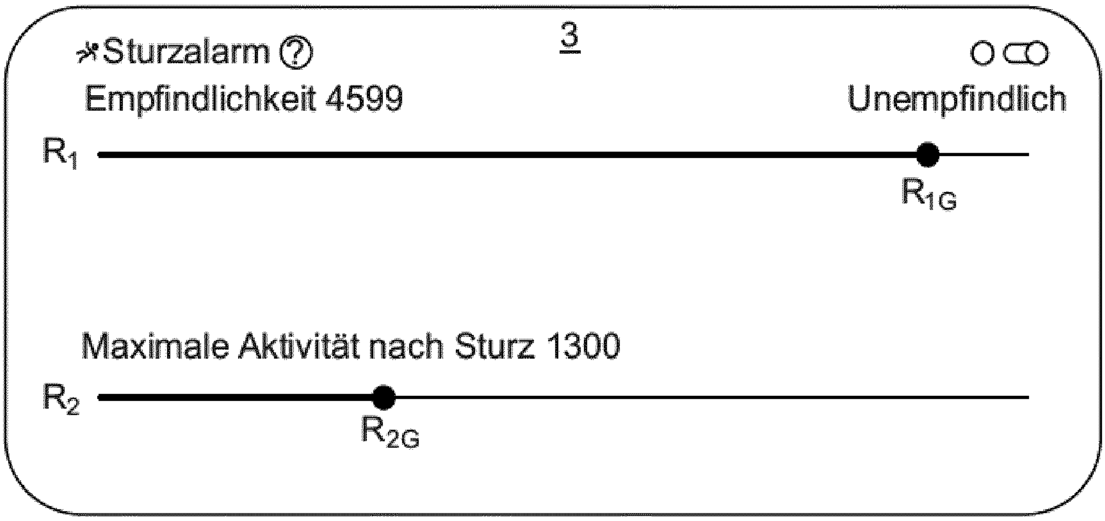
3
Sturzalarm
Empfindlichkeit 4599
Unempfindlich
$R_1$
$R_{1G}$
Maximale Aktivität nach Sturz 1300
$R_2$
$R_{2G}$

Fig. 2

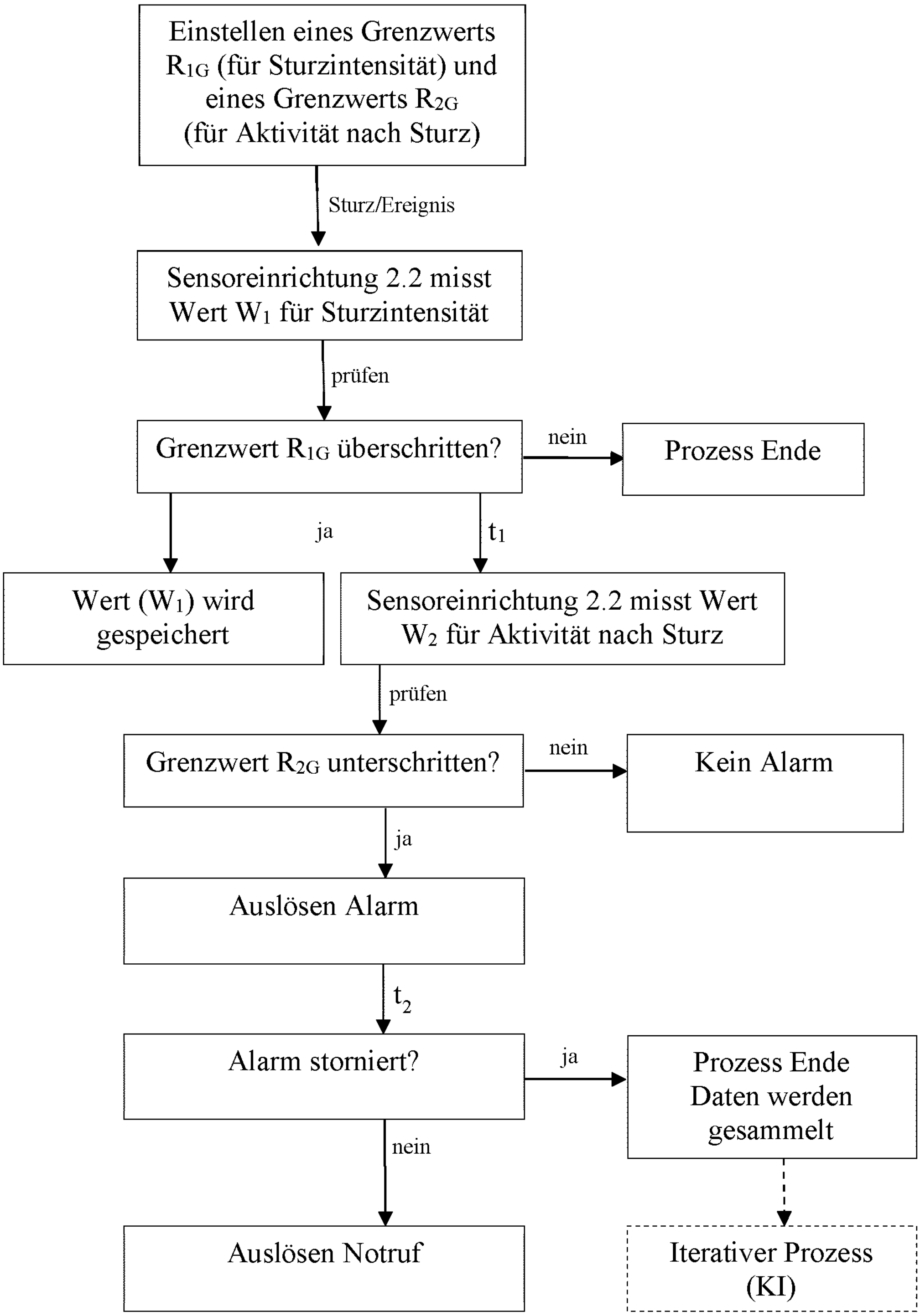
Einstellen eines Grenzwerts $R_{1G}$ (für Sturzintensität) und eines Grenzwerts $R_{2G}$ (für Aktivität nach Sturz)
Sturz/Ereignis
Sensoreinrichtung 2.2 misst Wert $W_1$ für Sturzintensität
prüfen
Grenzwert $R_{1G}$ überschritten?
nein
Prozess Ende
ja
$t_1$
Wert ($W_1$) wird gespeichert
Sensoreinrichtung 2.2 misst Wert $W_2$ für Aktivität nach Sturz
prüfen
Grenzwert $R_{2G}$ unterschritten?
nein
Kein Alarm
ja
Auslösen Alarm
$t_2$
Alarm storniert?
ja
Prozess Ende Daten werden gesammelt
nein
Auslösen Notruf
Iterativer Prozess (KI)

Fig. 3

Europäisches Patentamt
European Patent Office
Office européen des brevets

# EUROPÄISCHER RECHERCHENBERICHT

Nummer der Anmeldung
EP 24 16 4728

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2020/380840 A1 (GALARNEAU MICHELLE M [US] ET AL) 3. Dezember 2020 (2020-12-03)<br>* Abbildungen 1,3,4 *<br>* Absatz [0001] *<br>* Absatz [0020] *<br>* Absatz [0026] *<br>* Absatz [0030] *<br>* Absatz [0034] *<br>* Absatz [0044] *<br>* Absatz [0045] *<br>* Absatz [0046] *<br>* Absatz [0064] *<br>* Absatz [0066] *<br>* Absatz [0067] *<br>----- | 1-17 | INV.<br>G08B21/04 |
| A | WO 2021/032556 A1 (KONINKLIJKE PHILIPS NV [NL]) 25. Februar 2021 (2021-02-25)<br>* Absatz [0017] *<br>* Absatz [0021] *<br>* Absatz [0023] *<br>* Absatz [0025] *<br>* Absatz [0028] *<br>* Absatz [0031] *<br>* Absatz [0040] *<br>* Absatz [0047] *<br>* Absatz [0048] *<br>* Absatz [0049] *<br>* Absatz [0050] *<br>----- | 1-17 | RECHERCHIERTE SACHGEBIETE (IPC)<br>G08B |
| A | US 2016/038061 A1 (KECHICHIAN PATRICK [NL] ET AL) 11. Februar 2016 (2016-02-11)<br>* Abbildung 1 *<br>* Absatz [0048] *<br>* Absatz [0049] *<br>* Absatz [0050] *<br>* Absatz [0054] *<br>----- | 1-17 | |

Der vorliegende Recherchenbericht wurde für alle Patentansprüche erstellt

1

| Recherchenort | Abschlußdatum der Recherche | Prüfer |
|---|---|---|
| München | 9. April 2024 | Plathner, B |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR.

EP 24 16 4728

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

09-04-2024

| Im Recherchenbericht angeführtes Patentdokument | Datum der Veröffentlichung | Mitglied(er) der Patentfamilie | Datum der Veröffentlichung |
|---|---|---|---|
| US 2020380840 A1 | 03-12-2020 | CN 113891680 A | 04-01-2022 |
| | | EP 3975827 A1 | 06-04-2022 |
| | | US 2020380840 A1 | 03-12-2020 |
| | | US 2022246014 A1 | 04-08-2022 |
| | | WO 2020243061 A1 | 03-12-2020 |
| WO 2021032556 A1 | 25-02-2021 | CN 114269241 A | 01-04-2022 |
| | | EP 4018422 A1 | 29-06-2022 |
| | | JP 2022544757 A | 21-10-2022 |
| | | US 2021052198 A1 | 25-02-2021 |
| | | WO 2021032556 A1 | 25-02-2021 |
| US 2016038061 A1 | 11-02-2016 | AU 2014233947 A1 | 12-11-2015 |
| | | BR 112015023961 A2 | 18-07-2017 |
| | | CN 105051799 A | 11-11-2015 |
| | | EP 2976756 A1 | 27-01-2016 |
| | | JP 2016512777 A | 09-05-2016 |
| | | RU 2015145376 A | 27-04-2017 |
| | | US 2016038061 A1 | 11-02-2016 |
| | | WO 2014147496 A1 | 25-09-2014 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 20210275023 A1